# EUROPEAN PATENT APPLICATION

(11) **EP 1 669 441 A1**
(43) Date of publication of application: **14.06.2006**
(21) Application number: 04715554.4
(22) Date of filing: 27.02.2004
(51) Int. Cl.: C12N 5/10, A61K 35/28, A61K 35/407, A61P 1/16, A61P 43/00, C12N 15/54

(54) **METHOD OF DIFFERENTIATING MESENCHYMAL STEM CELL INTO LIVER CELL AND ARTIFICIAL HUMAN LIVER CELL**

(30) Priority: 27.08.2003 JP 2003303229
(71) Applicant: Renomedix Institute Inc., Ishikari-shi, Hokkai-do 0613241 (JP)
(72) Inventor: Hamada, Hirofumi, Sapporo-shi, Hokkaido 0640959 (JP); Niitsu, Yoshiro, Sapporo-shi, Hokkaido 0640823 (JP); Kato, Junji, c/o Sapporo Medical University, Sapporo-shi, Hokkaido 0608543 (JP); Sato, Yasushi, c/o Sapporo Medical University, Sapporo-shi, Hokkaido 0608543 (JP)
(74) Representative: Denison, Christopher Marcus
(86) International application number: PCT/JP2004/002440
(87) International publication number: WO 2005/024004

(57) **Abstract**

Establishment of hepatocytes is difficult. An object is to provide a means for preparing hepatocytes useful for hepatocyte transplantation therapy for liver injury, which comprises differentiating mesenchymal stem cells (the proliferation techniques of which have been established), and in particular, stroma cells, which are bone-marrow-derived mesenchymal stem cells, into hepatocytes. We have discovered that transplantation of human mesenchymal stem cells into the livers of mammals such as rats that have developed chronic liver injuries due to continuous administration of an agent can cause the human mesenchymal stem cells to differentiate into hepatocytes or mature hepatocytes. We have thus developed a means for differentiating mesenchymal stem cells into mature hepatocytes.

## Description

### TECHNICAL FIELD

The present invention relates to technology for differentiating mesenchymal cells into hepatocytes. The present invention further relates to technology concerning liver regenerative medicine for animals with liver injuries including humans with liver injuries.

### BACKGROUND ART

The liver is also called the "biochemical factory" of the human body. The liver is an important organ having various biochemical functions including intermediate metabolism involved in *in vivo* nutrition, biligenesis, generation and conversion of blood components, and detoxication, for example.

When liver injuries occur due to alcohol, a virus, a drug, an autoimmune disease, or the like, hepatitis and then hepatic cirrhosis are developed. This may have serious consequences.

For example, in the case of alcoholic liver injury, injuries occur in hepatocytes due to the chronic overconsumption of alcoholic beverages. Such injuries progress so that fatty liver, alcoholic hepatitis, and then terminal hepatic cirrhosis are developed. It is said that daily alcohol intake of between 60 g and 80 g or more has a high potential to result in fatty liver. It is also said that daily alcohol intake of between 100 g and 120 g or more for 10 years or longer results in hepatic cirrhosis.

Examples of conventional liver injury treatment include (1) treatment that involves elimination of poisonous substances that cause liver injury, such as alcohol; (2) treatment that involves supplying vitamins and the like and then waiting for natural regeneration of hepatocytes; (3) treatment that uses an agent such as (i) a glycyrrhizin pharmaceutical preparation (for protecting hepatic functions), (ii) Shosaikoto (for alleviating hepatic inflammation and improving hepatic functions), or (iii) a bile acid pharmaceutical preparation (useful for liver injury due to gallstone or bile stasis); (4) treatment that uses a remedy for viral hepatitis such as (i) interferon, (ii) Rebetol (an antiviral drug for use in combination with interferon; for chronic hepatitis C involving large viral amounts), or (iii) Lamivudine (an antiviral drug for hepatitis B and a DNA polymerase inhibitor); and (5) living donor liver transplantation and the like.

Non-patent document 1 reports that an alkaline phosphatase-positive rat stem cell-like cell line has been differentiated *in vitro* into a hepatocytic lineage. Non-patent document 2 reports differentiation of adult pluripotent cells into hepatocytes.

Furthermore, Patent document 1 discloses differentiation of bone marrow cells into hepatic parenchymal cells using a differentiation-inducing agent.

Patent document 1: JP Patent Publication (Kokai) 2002-78482 A

Non-patent document 1: Stem Cells Vol. 21, pp. 428-436

Non-patent document 2: The Journal of Clinical Investigation Vol. 109, pp. 1291-1302

### DISCLOSURE OF THE INVENTION

Drug therapies employed for hepatic treatment are only symptomatic therapies and are insufficient. However, it is difficult to establish hepatocytes and MAPCs (multipotent adult progenitor cells) required for cell transplantation therapy.

Hence, an object of the present invention is to provide a means for preparing hepatocytes useful for hepatocyte transplantation therapy for liver injury by differentiating mesenchymal stem cells for which proliferation techniques have been established (and in particular, stroma cells, which are bone-marrow-derived mesenchymal stem cells) into hepatocytes.

We have discovered that transplantation of mesenchymal stem cells, mesenchymal progenitor cells, or mesenchymal cells into the livers of mammals such as rats that have developed chronic liver injuries due to continuous administration of an agent causes these cells to differentiate into hepatocytes or mature hepatocytes. We have thus developed a means for differentiating mesenchymal stem cells, mesenchymal progenitor cells, or mesenchymal cells into mature hepatocytes.

According to the present invention, mature hepatocytes can be prepared using mesenchymal stem cells, mesenchymal progenitor cells, or mesenchymal cells that are available in sufficient quantities. Accordingly, for example, mesenchymal stem cells, mesenchymal progenitor cells, or mesenchymal cells prepared from individuals are differentiated into hepatocytes, so that individualized hepatocyte gene profiles can be produced. Such profiles can also be used for tailor-made medicine. Furthermore, hepatocytes can be prepared in large quantities, so that they can also be used for analyzing the proliferation mechanisms of hepatitis C virus and hepatitis B virus. Furthermore, these cells can also be used for analyzing the action mechanisms of anti-viral agents. Furthermore, normal mature human hepatocytes can be obtained in large quantities, although it has been impossible to conventionally obtain such cells. Hence, these cells can also be used for agent screening and agent toxicity tests. Furthermore, separated and harvested human hepatocytes can also be used for cell treatment that involves transplanting such cells into patients with liver failure and/or congenital metabolic liver disease. Furthermore, such hepatocytes can also be used for producing artificial livers.

This description includes part or all of the contents as disclosed in the description and/or drawings of Japanese Patent Application No. 2003-303229, which is a priority document of the present application.

### BRIEF DESCRIPTION OF THE DRAWINGS

Fig. 1 shows preparation of human mesenchymal stem cells.
Fig. 2 shows a method for administering allyl alcohol that induces chronic liver injury.
Fig. 3 shows antibody staining on day 14 after transplantation of human mesenchymal stem cells.
Fig. 4 shows antibody staining on day 28 after transplantation of human mesenchymal stem cells.
Fig. 5 shows the expression of human albumin in rat livers into which human mesenchymal stem cells have been transplanted.
Fig. 6 shows the expression of human α fetoprotein in rat livers into which human mesenchymal stem cells have been transplanted.
Fig. 7 shows the production amount of human albumin.

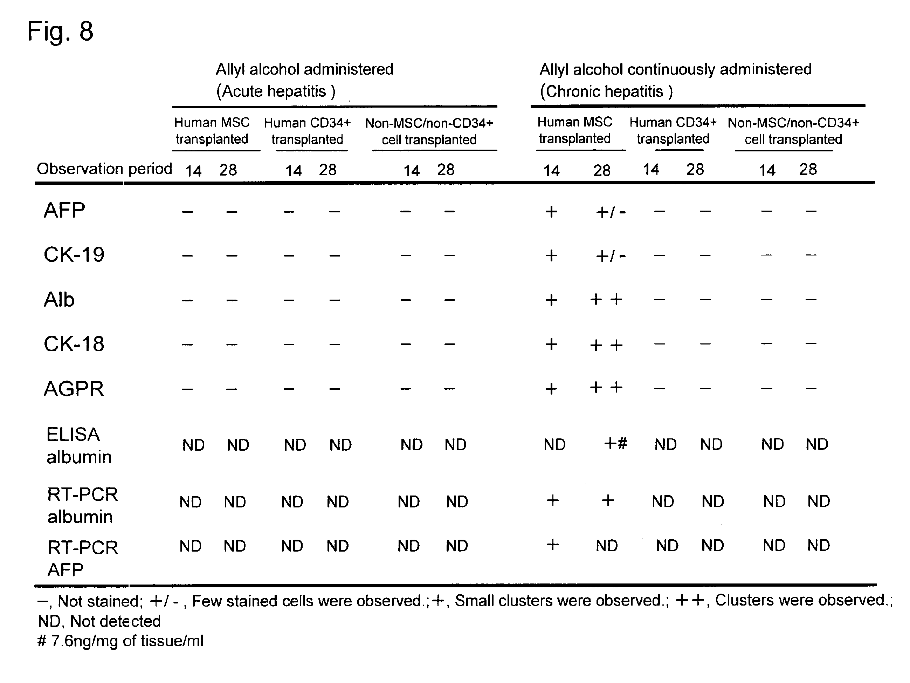

Fig. 8 shows the presence or the absence of differentiation of human mesenchymal stem cells, human CD34+ cells, or non-mesenchymal cells/non-CD34+ cells into human hepatocytes when allyl alcohol treatment has been performed once or continuously.
Fig. 9 shows hepatic differentiation of human mesenchymal hepatocytes (MSCs) into which TERT has been introduced (hepatic differentiation of TERT-introduced MSCs).

### BEST MODE OF CARRYING OUT THE INVENTION

The present invention provides a method for differentiating mesenchymal stem cells (also referred to as MSCs), mesenchymal progenitor cells, or mesenchymal cells into hepatocytes, which comprises transplanting mesenchymal stem cells, mesenchymal progenitor cells, or mesenchymal cells into mammals that have developed chronic liver injuries due to continuous administration of an agent, so as to cause these cells to differentiate into hepatocytes and mature hepatocytes.

The present invention also provides a method for causing chronic liver injury and for differentiating transplanted mesenchymal stem cells, mesenchymal progenitor cells, or mesenchymal cells into mature hepatocytes, which comprises transplanting mesenchymal stem cells, mesenchymal progenitor cells, or mesenchymal cells into mammals that have developed acute liver injuries and then continuously administering a liver-damaging agent to the mammals after transplantation.

### [Mesenchymal stem cells, mesenchymal progenitor cells, and mesenchymal cells]

As mesenchymal stem cells, mesenchymal progenitor cells, or mesenchymal cells, mesenchymal stem cells, mesenchymal progenitor cells, or mesenchymal cells derived from any mammal can be used. Preferable examples of such a mammal include humans and mice, rats, and monkeys that are generally used as experimental animals.

As mesenchymal stem cells, stem cells that can be obtained from bone marrow, peripheral blood, skin, hair roots, muscle tissues, endometrium, blood, and umbilical cord blood can be used, for example. Furthermore, stem cells that can be obtained from initial culture products of various tissues can be used. Furthermore, it is known that mesenchymal stem cells can also be separated from ES cells or teratoma cells. Here, examples of stem cells include totipotent stem cells that have totipotency and thus can differentiate into any cell type, pluripotent stem cells such as fetal stem cells that can differentiate into three germ layer lineages but differentiate in a limited way into extraembryonic trophoblastic cells, and multipotent stem cells that can differentiate into many cell types in a tissue. Mesenchymal stem cells are thought to be pluripotent.

Stem cells that can be used as mesenchymal stem cells are preferably prepared by performing initial culture of bone marrow and then obtaining target cells from interstitial cells that have adhered to the bottom surface of a culture dish.

"Progenitor cells of mesenchymal cells" means cells that have differentiated from mesenchymal stem cells and are on the way to differentiation into mesenchymal cells.

Mesenchymal cells are generated by differentiation from mesenchymal stem cells. Mesenchymal cells are incapable of differentiating into multiple types of cells as that are stem cells, but are capable of differentiating into a single type of cells and are capable of proliferation. Mesenchymal cells stay at the G0 phase under normal conditions, but can transfer to the G1 phase (start of division) when exposed to stimulation. Mesenchymal cells also include stroma cells and cells having the properties of stroma cells, for example. Mesenchymal cells are present in every tissue or organ including subcutaneous tissues, the lungs, and the liver, for example, and are specifically present in mesenchymal tissues such as bone, cartilage, fat, tendon, skeletal muscle, and bone marrow stroma.

Furthermore, in the present invention, the above mesenchymal stem cells, mesenchymal progenitor cells, or mesenchymal cells that have been immortalized can be used. These cells can be immortalized by the use of an immortalization gene, such as a telomerase gene or a gene that regulates the expression or the activity of telomerase, preferably human telomerase or a human telomerase catalytic subunit (hTERT).

Various known methods can be used as methods for introducing an immortalization gene into stroma cells, mesenchymal stem cells, or the like. Examples of such method that can be used herein include: a method that involves incorporating an immortalization gene into a plasmid vector and then introducing the vector into mesenchymal cells, mesenchymal stem cells (e.g., stroma cells), or the like in the presence of calcium-phosphoric acid so as to perform transformation; a method that involves causing an immortalization gene and a vesicle such as a liposome to come into contact with mesenchymal cells or mesenchymal stem cells, so as to introduce them into the cells; a method that involves performing introduction by electroporation in the presence of an immortalization gene; and a method that involves incorporating an immortalization gene into various virus vectors and then infecting mesenchymal cells, mesenchymal stem cells, or the like with the virus vectors, so as to perform introduction.

An example of an introduction method using a virus vector is an introduction method using a retrovirus, an adenovirus, or an adeno-associated virus. There is a method that uses a MoMLV virus as a retrovirus vector. Preferably, a pBabe vector can be used.

Furthermore, when immortalized cells are used for treating humans, it is thought that maximum removal of foreign genes such as an immortalization gene results in higher safety. Through previously established technology, an immortalization gene that has been introduced into a cell can be removed. For example, preferably, a technique that can be used herein involves specifically removing an immortalization gene sandwiched by loxP sequences or loxP-like sequences by treatment with recombinase such as Cre recombinase.

As human mesenchymal stem cells, preferably, mesenchymal stem cells derived from bone marrow can be used.

### [Subject animals for transplantation and means of causing chronic liver injury]

Mesenchymal stem cells will be explained as follows. Mesenchymal progenitor cells and mesenchymal cells can also be similarly differentiated into hepatocytes.

Mammals (hereinafter referred to as subject animals for transplantation) are used for providing tissues that have experienced chronic liver injuries, which provide an effective environment for differentiation of mesenchymal stem cells into hepatocytes. Various experimental animals can be used as such mammals. For example, preferably, mice, SCID mice, and rats, and particularly preferably SD rats, can be used. In addition, regarding rats, rats that are as young as possible in terms of weeks old are preferred. For example, 4- to 6-week-old rats are preferred.

Various drugs that can cause liver injury can be used as liver-damaging agents. For example, choline deficiency (CD) ethionine, galactosamine, dimethylnitrosamine (DMN), and Jo2 antibody (anti-Fas antibody; apoptosis induction) are known as liver-damaging agents. Preferably, allyl alcohol, carbon tetrachloride (CC14), or carbon tetrachloride (CCl4), and 2-acetylaminofluorene (2-AAF) can be used in combination. Alternatively, 2-AAF can be used after partial hepatectomy. Particularly preferably, allyl alcohol is desirably used. Allyl alcohol possesses a particularly damaging effect on hepatocytes in the vicinity of portal veins via necrosis.

When a mammal from which mesenchymal stem cells are derived is different from a subject mammal for transplantation, it is desired to previously administer an immunosuppressive agent to the subject animal for transplantation preferably 1 to 2 days before transplantation of mesenchymal stem cells, in order to reduce immune reaction upon transplantation.

As methods for transplantation to subject animals for transplantation, intrahepatic injection, intraportal vein injection, and caudal vein injection of a solution containing mesenchymal cells into the above subject animals can be employed, for example.

When chronic liver injury is caused through the use of a liver-damaging agent, transplant timings for mesenchymal stem cells are as follows. In view of the effects of a liver-damaging agent, cells can be transplanted into a subject animal at 24 hours at the earliest, within 3 days, and preferably 1 day after administration of the first liver-damaging agent.

In addition, cells can also be transplanted into livers already experiencing chronic liver injuries such as the livers of LEC rats constituting a Wilson's disease (cirrhosis) model and the livers of FAH-/- mice that can develop liver injuries due to hypertyrosinemia.

An example of a means for causing chronic liver injury in subject animals for transplantation is a method that involves transplanting mesenchymal stem cells into subject animals for transplantation and then continuously administering a liver-damaging agent, so as to cause chronic liver injury.

Concerning the dose of a liver-damaging agent, a dose sufficient for acutely damaging the liver while not affecting the survival of the animal can be selected for the first administration. For example, when allyl alcohol is used for rats, a dose approximately ranging from 0.5 mmol/kg to 0.7 mmol/kg and preferably a dose of approximately 0.6 mmol/kg can be employed. In the second and the following administrations, a dose sufficient for a liver-damaging agent to cause chronic liver injury can be employed. For example, when allyl alcohol is used, a dose of 0.3 mmol/kg can be employed.

As a route of administration of a liver-damaging agent, an administration method preferable for each agent can be selected. For example, a liver-damaging agent can be administered via oral administration, intravenous injection, or intraperitoneal injection. When allyl alcohol is administered to rats, it can be administered preferably via intraperitoneal injection.

Regarding the administration period for a liver-damaging agent, the agent is administered during a period sufficient to cause the development of chronic liver injury. Preferably, such a period is a period required for differentiation of mesenchymal stem cells into mature hepatocytes. For example, a liver-damaging agent can be administered during a period ranging from 2 weeks to 3 months, preferably a period ranging from 3 weeks to 5 weeks, and further preferably a period of 4 weeks. For example, when allyl alcohol is used as a liver-damaging agent and human mesenchymal stem cells are transplanted into rats, intraperitoneal injection of a dose of 0.3 mmol/kg can be performed 3 times a week and this can be continued for 1 month.

### [Confirmation of differentiation into hepatocytes]

Examples of markers (labels) for undifferentiated hepatocytes (immature hepatocytes) include GST-P, cytokeratin 19, and α-phetoprotein. To detect these markers, antibodies against these markers or primers or probes specific to genes encoding these markers can be used. Alternatively, furthermore, undifferentiated hepatocytes (immature hepatocytes) can be discriminated by γ-GTP staining.

Examples of markers (labels) for mature hepatocytes include albumin, α1-antitrypsin, transferrin, CK18, and AGPR. These markers can be detected by the use of antibodies against these markers or primers or probes specific to genes encoding these markers.

Undifferentiation or maturity can be examined through comparison of reactivities to an anti-albumin antibody, an anti-AFP (αfetoprotein) antibody, an anti-CK19 antibody (where "CK" is an abbreviation for cytokeratin, and the same applies in the following description), an anti-CK18 antibody, an anti-AGPR antibody (where "AGPR" is an abbreviation for an asialoglycoprotein receptor, and the same applies in the following description), and the like.

Furthermore, when mesenchymal stem cells are transplanted into the livers of animals that are different from the animals from which the cells are derived, and specifically, when human mesenchymal stem cells are transplanted into rat livers that have experienced liver injuries, for example, differentiation can be confirmed through an immunostaining method, an immunoenzyme staining method, a sandwich antibody method, or the like using antibodies specific to animals from which mesenchymal cells are derived and also specific to the above mature hepatocyte markers or antibodies specific to human albumin.

Furthermore, when primers specific to markers are used, a gene amplification method such as PCR can be used.

### [Harvest of hepatocytes obtained by differentiation]

As an example, livers are excised from experimental animals and then hepatocytes are separated from the livers. The hepatocytes are treated with fluorescent-labeled antibodies specific to animals from which mesenchymal cells are derived and also specific to mature hepatocytes or antibodies against cell surface markers (e.g., HLA). Mature hepatocytes that have differentiated from mesenchymal stem cells can be isolated using a fluorescence activated cell sorter (FACS).

Human telomerase is introduced into MSCs, thereby obtaining immortalized TERT-introduced MSCs. Such TERT-introduced MSCs are caused to differentiate into the liver by the above method. The cells separated by perfusion are cultured. Rat-derived hepatocytes generally die within approximately 1 week. Accordingly, the cells that have survived are mature hepatocytes that have differentiated from mesenchymal stem cells. These cells are cultured and proliferated in appropriate media.

### [Use of hepatocytes obtained by differentiation]

Hepatocytes are prepared from individuals by differentiating mesenchymal stem cells and then harvested. Individualized hepatocyte gene profiles can be produced using the harvested hepatocytes. Such profiles can also be applied for tailor-made medicine. Furthermore, hepatocytes can be prepared in large quantities, so that they can also be used for the analysis of the proliferation mechanisms of hepatitis C virus and hepatitis B virus and the analysis of the action mechanism of anti-viral agents. Moreover, normal mature human hepatocytes that have conventionally been impossible to obtain can be obtained in large quantities. Thus, such normal mature human hepatocytes can also be used for agent screening and agent toxicity tests.

Furthermore, separated and harvested human hepatocytes can also be used for cell treatment that involves transplantation of such hepatocytes into patients with liver failure and/or congenital metabolic liver disease or for production of artificial livers.

### [Cell treatment agent using mesenchymal stem cells]

Furthermore, a mesenchymal stem cell can also be used as a cell treatment agent that is directly administered to a hepatic lesion together with an appropriate carrier in cell treatment for chronic hepatitis.

The present invention will be explained in greater detail with reference to examples. The examples are merely embodiments of the present invention. The present invention is not limited by these examples.

### Example 1

### [Differentiation of mesenchymal stem cells into hepatocytes]

### 1-1 Mesenchymal stem cell (MSC):

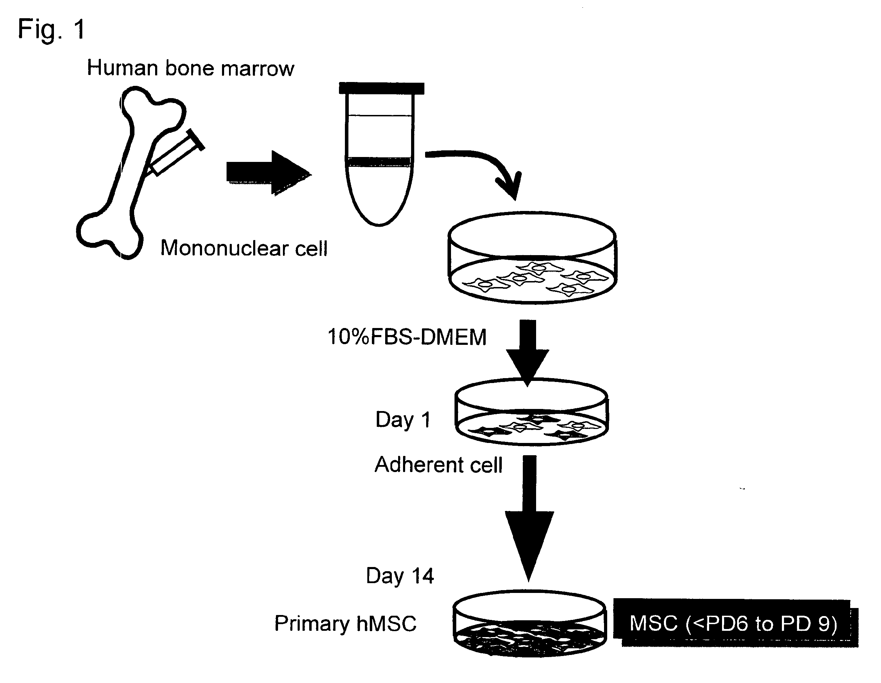

Bone marrow aspiration was performed and samples were obtained from the iliac bone of a healthy adult. Mononuclear cells were harvested by gradient centrifugation and cultured overnight in DMEM containing 10% inactivated fetal bovine serum. Culture of adherent cells was initiated on the next day. Cells were harvested with T-E (trypsin-EDTA) 2 weeks later and then cryopreserved as primary mesenchymal stem cells. The mesenchymal stem cells of the primary culture were successively cultured in DMEM containing 10% inactivated fetal bovine serum. The cells that had successively divided from the primary culture through PD (population doubling) 6 to PD9 (6^{th} through 9^{th} generations) were used for the following experiments (Fig. 1).

### 1-2 Experimental animal:

Spragne-Dawley (SD) rats (5-week-old, female or male) purchased from Charles River Japan, Inc. were used.

### 1-3 Liver injury model and MSC administration method:

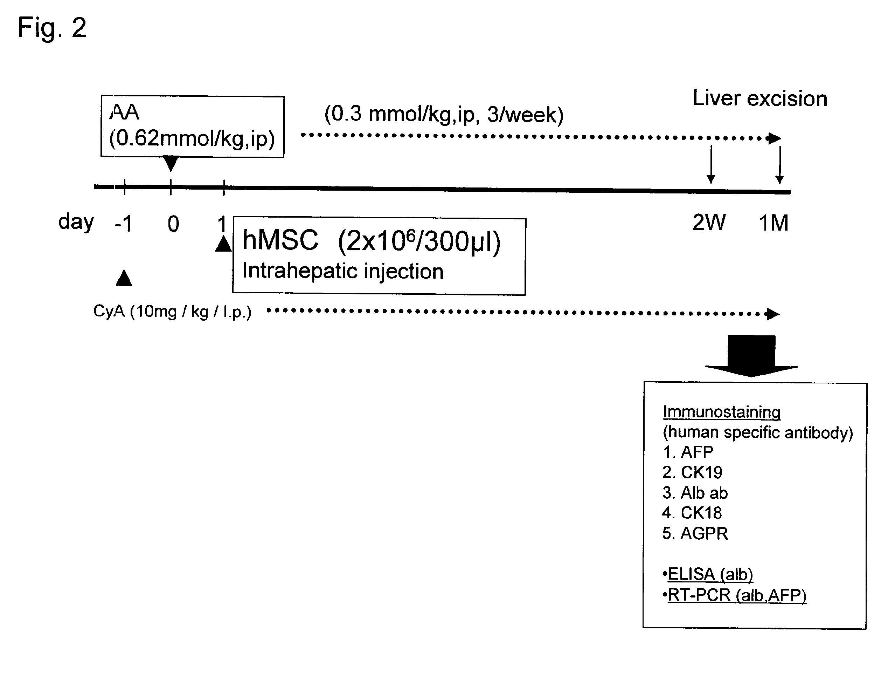

2 days before MSC administration (day -1), intraperitoneal administration of cyclosporin A, (CyA, Sandimmun: purchased from Novartis Pharma K.K.) was started at 10 mg/kg/day. On the next day (day-0), allyl alcohol (AA) (0.62 mmol/kg) was intraperitoneally administered based on the report of Yavorkovsky et al. (Hepatology 1995; 21: 1702-1712) to cause liver injury. 2 days (day 1) after day -1, MSCs (mesenchymal stem cells) adjusted at a concentration of 2x10⁶ cells/300 µl were injected directly by local injection to rat livers using a 23G syringe. Subsequently, chronic liver injury was caused by 3-times-a-week administration of AA (0.3 mmol/kg) (Fig. 2).

### 2 Confirmation of differentiation into hepatocytes

Differentiation into human hepatocytes was confirmed by 1) staining with a human-specific and liver-specific marker, 2) detecting human-specific albumin (Alb) and α phetoprotein (AFP) mRNA, and 3) detecting human-specific albumin protein production by the ELISA method.

### 2-1 Staining with human-liver-specific marker:

Rats were sacrificed on day 14 and on day 28 after MSC administration. The livers were fixed by perfusion with 4% paraformaldehyde and then embedded with an OTC compound. Thus, frozen sections (6 µm) were prepared. These sections were subjected to immunostaining using an anti-human Alb (albumin) antibody (Sigma-Aldrich Co., A6684), an anti-human AFP antibody (Sigma-Aldrich Co., A8452), an anti-human CK19 antibody (Sigma-Aldrich Co., C6930), an anti-human CK18 antibody (PROGEN, RCK106), and an AGPR antibody (Kohgo et al., Hybridoma 1993; 12. 591-598), which had been previously confirmed not to cross-react with rat liver tissues, a control antibody, and dyes.

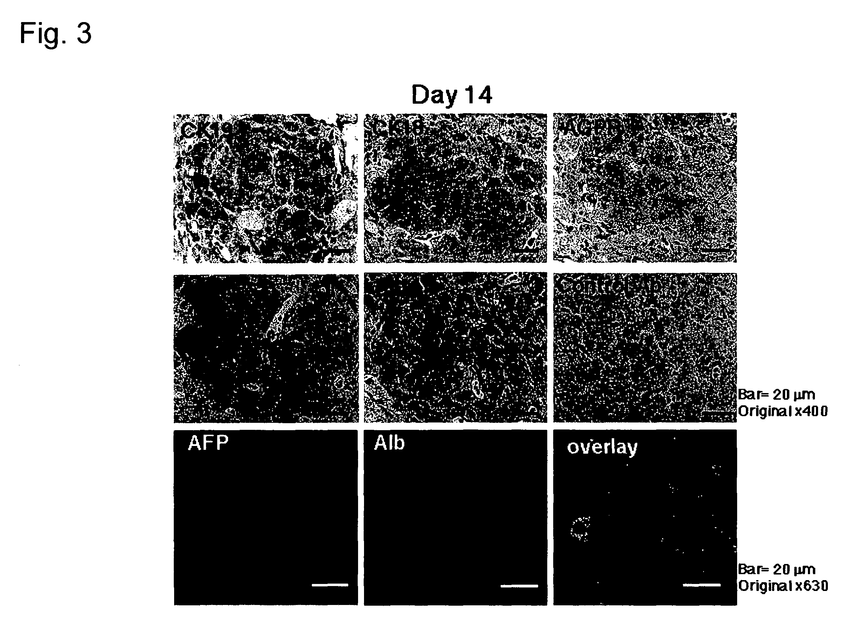

Fig. 3 shows the results on day 14 after MSC transplantation at 400 times magnification. As shown in the upper and the middle columns in Fig. 3, approximately 50 to 80 cell clusters (approximately 20 µm to 25 µm in diameter) positive for the anti-human AFP antibody, the anti-human CK19 antibody, the anti-human Alb antibody, the anti-human CK18 antibody, or the anti-AGPR antibody were observed in the same section. No staining was observed in the case of the control antibody. As shown in fluorescent immunostaining in the lower column , the cells were stained with rhodamine-labeled anti-human AFP antibody (left side, shown red) or with FITC-labeled anti-human Alb antibody (center, shown green). Such cells were observed to be yellow in the merged image thereof (overlay, shown on the right). Hence, it was considered that these transplanted MSCs expressed AFP and Alb. These staining patterns indicate that such cells were relatively immature human hepatocytes.

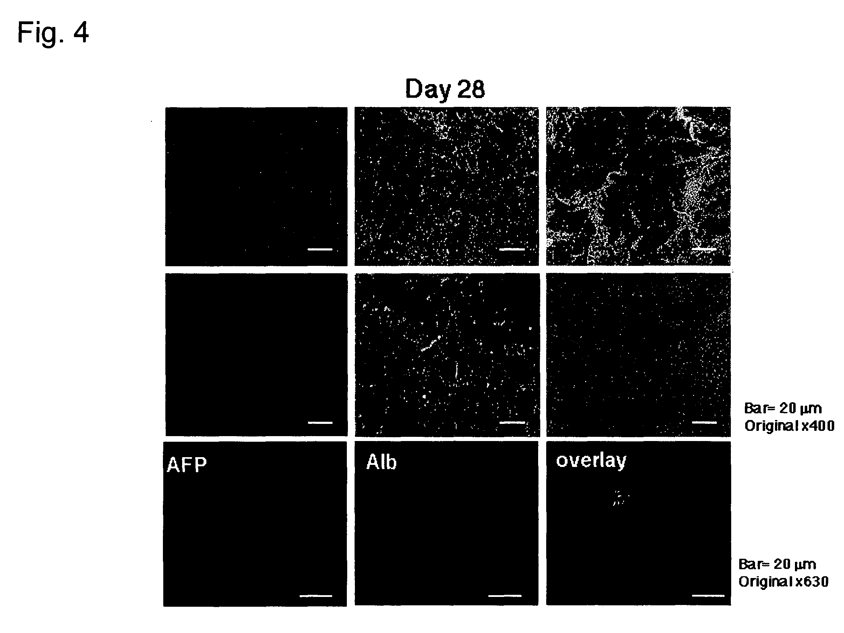

Fig. 4 shows the results of immunostaining on day 28 after MSC transplantation. 400 times magnification was similarly employed as in the case on day 14 after MSC transplantation. As shown in the upper and the middle columns, 300 or more cell clusters (25 µm to 30 µm in diameter) positive for the anti-human Alb antibody, the anti-human CK18 antibody, or the AGPR antibody were observed in the same section. In contrast, only a few cells positive for the anti-human AFP antibody or the anti-human CK19 antibody were occasionally observed. As shown in fluorescent immunostaining in the lower column, cells (similarly shown red) positive for the rhodamine-labeled anti-human AFP antibody were partially observed and cells were entirely stained in the case of the FITC-labeled anti-human Alb antibody (center). As shown in the merged image shown on the right, AFP antibody-positive cell portions were observed to be yellow. Hence, it was considered that most of these transplanted MSCs expressed Alb, but AFP expression partially remained. These staining patterns indicate human mature hepatocytes.

### 2-2 Detection of human-specific albumin (Alb) and α phetoprotein (AFP) mRNA

RNA was extracted from liver tissue and then cDNA was prepared using reverse transcriptase. PCR was performed for human-specific Alb and AFP using the cDNA as a template and corresponding primers. Band amplification was confirmed by agarose gel electrophoresis.

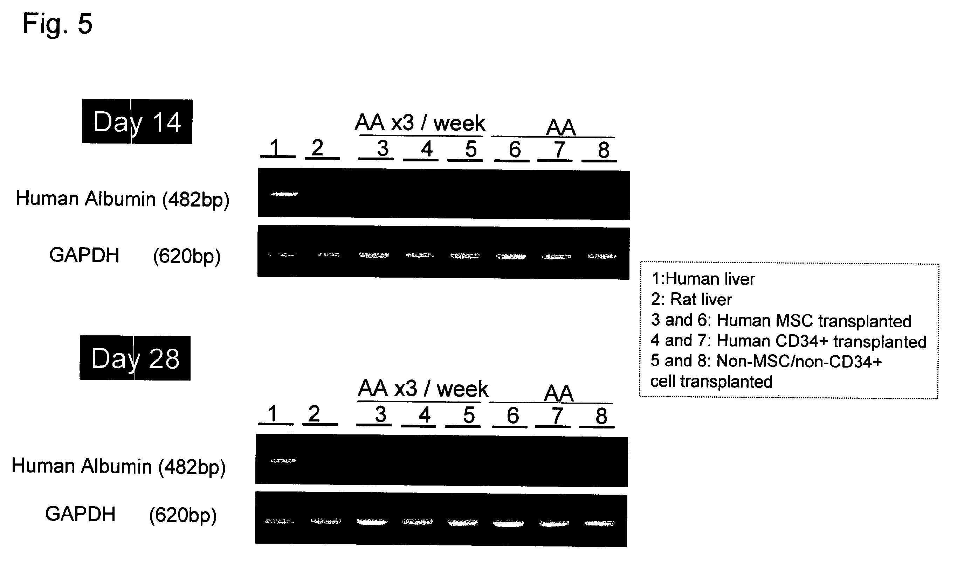

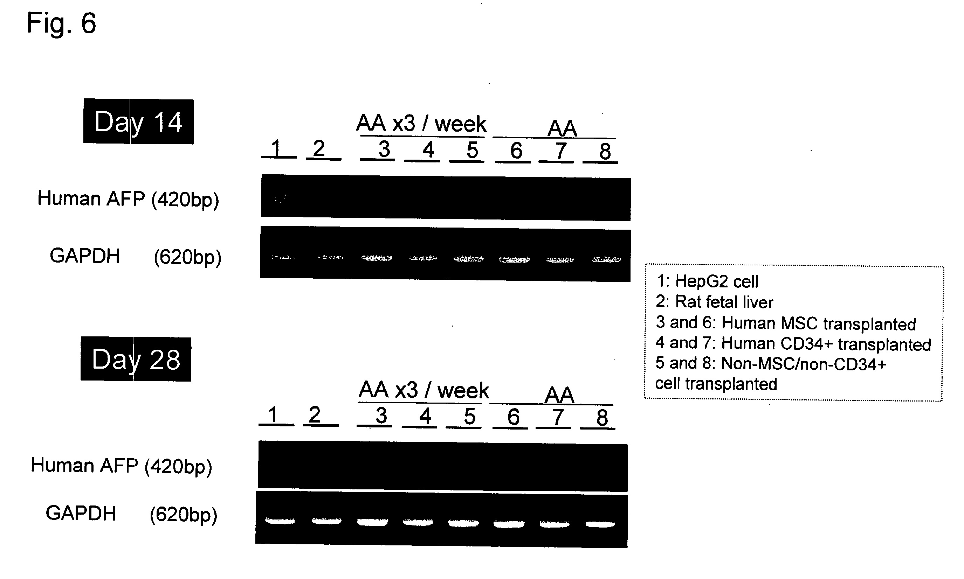

Regarding primer sequences, 5'-cttcgtctgccaaacagagactca-3' (24 mer) was used as a primer sense strand (Hu-Albumin(S)) for human albumin, 5'-acagagtaatcaggatgccttcttg-3' (25 mer) was used as a primer antisense strand (Hu-Albumin(AS)) for human albumin, 5'-ttggagaagtacggacattcagact-3' (25 mer) was used as a primer sense strand (Hu-AFP(S)) for human α fetoprotein, and 5'-gactcagtttagtaacagttatggct-3' (26 mer) was used as a primer antisense strand (Hu-AFP(AS)) for human α fetoprotein. DNA samples were amplified by denaturation at 94°C for 5 minutes, followed by 40 reaction cycles each consisting of 94°C for 1 minute (denaturation), 65°C for 1 minute (annealing), and 72°C for 30 seconds (extension). Amplification products were visualized by ethidium bromide staining on 2.5% agarose gel electrophoresis. Thus, a band of 482 bp was observed in the case of Alb and a band of 420 bp was observed in the case of AFP (Figs. 5 and 6).

Fig. 5 shows the results of examining Alb expression: 1) HepG2 cells (positive control) that were liver cancer cells; 2) rat liver (negative control); 3) AA was administered 3 times a week after intrahepatic injection of MSCs; 4) AA was administered 3 times a week after intrahepatic injection of control CD34+ cells; 5) AA was administered 3 times a week after intrahepatic injection of non-MSCs/non-CD34+ cells (excluding control MSCs and CD34); 6) AA was administered only once after intrahepatic injection of MSCs; 7) AA was administered only once after intrahepatic injection of control CD34+ cells; and 8) AA was administered only once after intrahepatic injection of non-MSCs/non-CD34+ cells (excluding control MSCs and CD34). On day 14 and on day 28 after MSC administration, the human Alb band was observed only in the case where AA had been administered 3 times a week after intrahepatic injection of MSCs.

Fig. 6 shows the results of examining AFP expression: 1) HepG2 cells (positive control) that were liver cancer cells; 2) rat fetal liver (negative control); 3) AA was administered 3 times a week after intrahepatic injection of MSCs; 4) AA was administered 3 times a week after intrahepatic injection of control CD34+ cells; 5) AA was administered 3 times a week after intrahepatic injection of non-MSCs/non-CD34+ cells excluding control MSCs and CD34; 6) AA was administered only once after intrahepatic injection of MSCs; 7) AA was administered only once after intrahepatic injection of control CD34+ cells; and 8) AA was administered only once after intrahepatic injection of non-MSCs/non-CD34+ cells excluding control MSCs and CD34. On day 14, the human AFP band was observed only in the case where AA had been administered 3 times a week after intrahepatic injection of MSCs, but no such bands were observed on day 28.

### 2-3 Detection of human-specific albumin protein production through ELISA method

Next, to confirm whether or not the transplanted human MSCs actually produced human Alb, that is, whether the MSCs were functional or not, 10 mg of the liver tissue into which MSCs had been injected by local injection was collected. The homogenate thereof was then examined using a human albumin ELISA kit (Bethyl Laboratories, Montgomery, Texas) capable of detecting human-specific Alb protein.

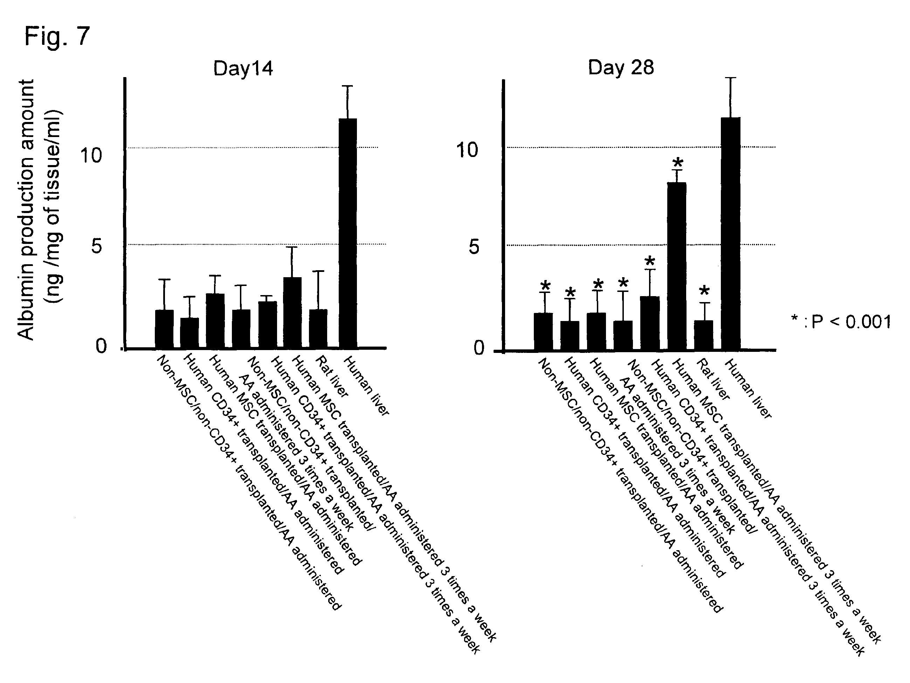

Fig. 7 shows the results of examining Alb production: from right to left in order,
1) human liver tissue (positive control); 2) rat liver (negative control); 3) AA was administered 3 times a week after intrahepatic injection of MSCs; 4) AA was administered 3 times a week after intrahepatic injection of control CD34+ cells; 5) AA was administered 3 times a week after intrahepatic injection of non-MSCs/non-CD34+ cells excluding control MSCs and CD34; 6) AA was administered only once after intrahepatic injection of MSCs; 7) AA was administered only once after intrahepatic injection of control CD34+ cells; and 8) AA was administered only once after intrahepatic injection of non-MSCs/non-CD34+ cells excluding control MSCs and CD34.

In the case of rats with liver injuries, into which MSCs had been transplanted and to which AA had been continuously administered, human Alb production was significantly increased on day 14 after transplantation to 4 ng/mg of tissues/ml and significantly increased on day 28 after transplantation to 7.6 ng/mg of tissue/ml in the liver tissue, compared with the other controls. Thus, human Alb production at levels analogous to that in the case of the human liver tissue was confirmed.

### 3. Conclusion

In the case of liver injury caused by the use of allyl alcohol (AA), hepatic differentiation of MSCs was shown not in the acute hepatitis group, but in the chronic liver injury models to which AA had been administered 3 times a week. This may indicate that sustained liver injury due to AA is an environment appropriate for inducing differentiation of MSCs into the liver.

On the other hand, in the systems to which bone marrow fractions other than MSCs had been injected by local injection, differentiation to the liver was not observed in any liver injury models. As reported so far (Wang X et al., Am J Pathol 2002; 161: 565-574), such results are considered to support the fact that bone marrow ablation through radiation, so as to cause engraftment of transplanted cells to the bone marrow is essential for hepatic differentiation using HSCs (hematopoietic stem cells) or bone marrow cells.

### Example 2

### Hepatic differentiation of TERT-introduced MSC

Based on the method of Kawano et al., (Blood. 2003; 101, 532-40), MSCs (TERT-MSCs) wherein prepared hTERT had been introduced were prepared using the method described in the following reference example 1. Observation was performed on day 28 after the intrahepatic injection performed by the method of Example 1.

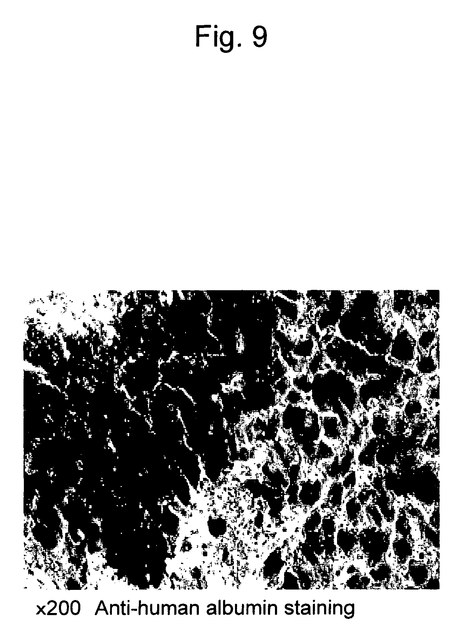

As a result, as shown in Fig. 9, cells expressing human albumin were confirmed in normal rat liver tissues. It was considered that these cells had differentiated into mature hepatocytes.

### Example 3

### Separation by perfusion of hepatocyte-like cells derived from mesenchymal stem cells

(1) Rats (Example 2) into which hTERT-MSCs derived from hTERT-gene-introduced mesenchymal stem cells had been intrahepatically injected were subjected to hair shaving and laparotomy on day 28 after the local injection under deep anesthesia. Perfusion was performed from the portal vein with an EGTA solution (prepared using 500 ml of a Hanks solution + 1.19 g of HEPES + 0.1 g of EGTA) at 37°C. After confirmation of the swelling of the livers, upper and lower inferior vena cavas of the livers were cut. Perfusion was performed at a rate of 10 ml/min for 10 minutes. Next, perfusion was performed with a collagenase solution at 37°C (500 ml of a Hanks solution + 0.235 g of CaCl2/2H2O + 1.19 g of HEPES + 100 mg (100 U/ml) of collagenase (collagenase Yakult)) at a rate of 10 ml/min for 10 minutes. After confirmation of the expression of white spots on the liver surface, sites at which hTERT-MSCs had been injected by local injection were cut with a clamp. The resultants were finely cut within a cooled Hanks solution using forceps and a clamp and then filtered with 100 µm and 70 µm filters. Subsequently, each resultant was centrifuged for 1 minute at 50 g, the supernatant was removed, the resultant was suspended again within a cooled Hanks solution, and then each resultant was centrifuged for 1 minute at 50 g. This was repeated 3 times. Cell number was then counted. As a result, approximately 2x10⁸ cells were harvested.
(2) Culture of hepatocyte-like cells derived from mesenchymal stem cells
   Subsequently, the cells obtained by the above perfusion were cultured. The cells were inoculated at 1.4×10⁴ cell/cm² on an L15 medium (0.2% BSA, 50 mg/l gentamicin, 100 nM dexamethasone, and 0.5 mg/l insulin had been added to 500 ml of L15). 3 hours later, the medium was replaced by an HCM medium (HCM Bullet Kit (TAKARA BIO INC.)) and then the cells were cultured within a CO2 incubator at 37°C.
   Rat hepatocytes die within approximately 1 week. The cells were observed for approximately 1 week. The cells forming colonies were hTERT-MSC-derived human hepatocytes.

### [Reference example 1]

### Preparation of hTERT-MSC

### 1. Separation of mesenchymal stem cells

Bone marrow aspiration was performed and then samples were obtained from the iliac bone of a healthy adult. Mononuclear cells were collected by a gradient centrifugation method and then cultured overnight in DMEM containing 10% inactivated fetal bovine serum. Culture of adherent cells was initiated on the next day. The cells were harvested with T-E (trypsin-EDTA) 2 weeks later and then cryopreserved as the primary mesenchymal stem cells.

### 2. hTERT

As a gene to be introduced into mesenchymal stem cells, a gene encoding a human telomerase catalytic activity subunit (hTERT) was used. The hTERT sequence is described in Science 277, pp. 955-959, for example.

### 3. Vector used for gene transfer into mesenchymal stem cells (Fig. 1)

pBABE-hygro-hTERT (provided by Dr. Robert A. Weinberg) was prepared by cloning a hTERT EcoRV-SalI fragment (obtained by PCR from pCI-Neo-hTERT-HA) into pBABE-hygro as described in Proc. Natl. Acad. Sci. U.S.A. vol. 95, pp. 14723-14728.

### 4. Preparation of retrovirus-producing cells and viral infection using the same were performed according to "Experimental Medicine, Separate Volume, The Protocol Series, Experimental Methods for Gene Transfer & Expression Analysis (Idenshi Donyu & Hatsugen Kaiseki Jikken Ho) (edited by Izumi Saito and Sumio Sugano, YODOSHA CO., LTD., pp. 58-62)."

Specifically, Ψ CRIP packaging cells (Proc. Natl. Acad. Sci. U.S.A. 90: 3539-3543, 1993) were prepared using BOSC23 packaging cells (Proc. Natl. Acad. Sci. U.S.A., 90: 8392-8396, 1993) as described below.

### 4-1. Preparation of recombinant-retrovirus-vector-producing cells

(i) 5.5x10⁶ BOSC23 cells were inoculated on a 10 cm dish 18 to 24 hours before transfection.
(ii) 800 µl of OPTI-MEM (Gibco/BRL) was gently added to 15 µg of DNA (retrovirus vector) and then the resultant was stirred, thereby preparing an A solution.
(iii) 750 µl of OPTI-MEM was collected in a sterilized tube. 50 µl of LIPOFECTAMINE (2 mg/ml Gibco/BRL) was added to the tube and then the resultant was slowly mixed, thereby preparing a B solution.
(iv) The A solution was gently mixed with the B solution, thereby preparing a C solution. The C solution was allowed to stand at room temperature for 30 to 45 minutes.
(v) The BOSC23 cells were washed once with a medium at 37°C from which an antibiotic agent and FBS had been removed.
(vi) The C solution (1.6 ml) was gently added to the BOSC23 cells.
(vii) 2.4 ml of OPTI-MEM was then added.
(viii) The resultant was incubated for 5 hours under 5% CO2.
(ix) 4 ml of DMEM containing 20% fetal bovine serum was added to the resultant and then the resultant was incubated overnight.
(x) The medium was replaced by a medium at 37°C containing 10% fetal bovine serum and 1 to 2x10⁶ ΨCRIP packaging cells were inoculated on a 10 cm dish simultaneously.
(xi) 24 hours later, the medium for the BOSC23 cells was filtered with a 0.45 µm or 0.20 µm syringe filter. The medium for ΨCRIP was replaced by 5 ml of a filtered medium. Simultaneously, polybrene (Hexadimethrine Bromide, SIGMA H-9268) was added to a concentration of 8 µg/ml.
(xii) After 4 to 24 hours of culture, 5 ml of a medium was added and then the cells were further cultured overnight.
(xiii) Agent selection was performed and then retrovirus-producing Ψ CRIP cells were prepared.

Next, the above vector was proliferated in the retrovirus-producing cells (φ CRIP/P131) and then mesenchymal stem cells were infected (gene transfer) with the vector as described below.

First, on the day before infection, mesenchymal stem cells were re-inoculated to a concentration of 5x10⁴ cells/10 cm dish. The medium (DMEM containing 10% bovine serum) for retrovirus-producing φ CRIP/P 131 was replaced by an α-MEM medium containing 12.5% inactivated equine serum and 12.5% inactivated fetal bovine serum/2-Mercaptoethanol/hydrocortisone. The cells were then cultured. On the day of infection, a culture supernatant was filtered with a 0.20 µm filter and then polybrene was added to a final concentration of 8 µg/ml. Next, mesenchymal stem cells were infected with recombinant retrovirus vectors produced in the supernatant. 4 hours later, the culture supernatant was replaced by a new medium, followed by another 2 days of culture. Subsequently, pBABE-hygro-hTERT was subjected to 5 days of agent selection with 100 µg/ml hygromycin.

The cells were infected with retrovirus vectors, specifically with (1) a control or (2) the pBABE-hygro-hTERT vector only.

In addition, these viruses and cells are kept by the present inventors and are ready for distribution at any time after patent obtainment.

All publications, patents, and patent applications cited herein are incorporated herein by reference in their entirety.

### Industrial Applicability

The present invention can be used in industrial fields including cell differentiation, cell culture, pharmaceutical development, artificial organ development, and the like. Furthermore, the present invention can also be used in industrial fields relating to technology for liver regenerative medicine.

## Claims

1. A method for differentiating a mesenchymal stem cell, a mesenchymal progenitor cell, or a mesenchymal cell into a hepatocyte using liver tissue that has experienced chronic livery injury.

2. The method according to claim 1, wherein the liver tissue that has experienced chronic liver injury is developed in a mammalian liver by long-term administration of a hepatocyte-damaging agent to a mammal.

3. A method for differentiating a mesenchymal stem cell, a mesenchymal progenitor cell, or a mesenchymal cell into a hepatocyte, which comprises the following steps (1) to (3) of:
(1) administering a hepatocyte-damaging agent to a mammal;
(2) transplanting a mesenchymal cell, a mesenchymal progenitor cell, or a mesenchymal cell into the liver of the relevant mammal; and
(3) administering a hepatocyte-damaging agent continuously to the relevant mammal.

4. The method according to any one of claims 1 to 3, wherein the mesenchymal stem cell, the mesenchymal progenitor cell, or the mesenchymal cell is a mesenchymal stem cell derived from a human.

5. The method according to any one of claims 1 to 4, wherein the mesenchymal stem cell is a mesenchymal stem cell derived from a human.

6. The method according to any one of claims 1 to 5, wherein the mesenchymal stem cell is a mesenchymal stem cell in which hTERT (human telomerase catalytic activity subunit) has been introduced.

7. The method according to any one of claims 2 to 6, wherein the hepatocyte-damaging agent is allyl alcohol.

8. The method according to any one of claims 2 to 7, wherein an immunosuppressive agent is administered before transplantation of the mesenchymal stem cell, the mesenchymal progenitor cell, or the mesenchymal cell.

9. The method according to any one of claims 2 to 8, wherein the mesenchymal cell, the mesenchymal progenitor cell, or the mesenchymal cell was intrahepatically injected.

10. The method according to any one of claims 2 to 9, wherein the mammal is a rat.

11. A method for discriminating with a label specific to a hepatocyte derived from a mesenchymal stem cell and harvesting such hepatocyte obtained as a result of differentiation caused by the method according to any one of claims 1 to 10.

12. A hepatocyte obtained as a result of differentiation caused by the method according to any one of claims 1 to 10.

13. A therapeutic agent for liver injury, which contains as an active ingredient a hepatocyte obtained by differentiation of a mesenchymal stem cell, a mesenchymal progenitor cell, or a mesenchymal cell.

14. A therapeutic agent for liver injury, which contains as an active ingredient a mesenchymal stem cell, a mesenchymal progenitor cell, or a mesenchymal cell.


SEQUENCE LISTING

<110> Renomedix Institute Inc.

<120> Method of differentiating Mesenchymal Stem cell into hepatocyte and
artificial human hepatocyte

<130> PH-2076-PCT

<140>
<141>

<150> JP 2003-303229
<151> 2003-08-27

<160> 4

<170> PatentIn Ver. 2.1

<210> 1
<211> 24
<212> DNA
<213> Artificial Sequence

<220>
<223> Description of Artificial Sequence:primer

<400> 1
cttcgtctgc caaacagaga ctca                                        24

<210> 2

<211> 25

<212> DNA

<213> Artificial Sequence

<220>

<223> Description of Artificial Sequence:primer

<400> 2

acagagtaat caggatgcct tcttg                                                25

<210> 3

<211> 25

<212> DNA

<213> Artificial Sequence

<220>

<223> Description of Artificial Sequence:primer

<400> 3

ttggagaagt acggacattc agact                                                25

<210> 4

<211> 26

<212> DNA

<213> Artificial Sequence

<220>
<223> Description of Artificial Sequence:primer

<400> 4
gactcagttt agtaacagtt atggct                                          26